# EUROPÄISCHE PATENTSCHRIFT

(11) **EP 0 391 203 B1**
(45) Veröffentlichungstag und Bekanntmachung des Hinweises auf die Patenterteilung: **07.12.1994**
(21) Anmeldenummer: 90105749.7
(22) Anmeldetag: 26.03.1990
(51) Int. Cl.: C07D 405/02, C09K 19/34

(54) **2-Phenylpyridine**
2-Phenyl pyridins
Phényl-2 pyridines

(30) Priorität: 04.04.1989 CH 1238/89
(43) Veröffentlichungstag der Anmeldung: 10.10.1990
(73) Patentinhaber: F. HOFFMANN-LA ROCHE AG, 4002 Basel (CH)
(72) Erfinder: Leenhouts, Frans, Dr., B-3590 Achel (BE); Villiger, Alois, Dr., CH-4055 Basel (CH)
(74) Vertreter: Cottong, Norbert A.

(56) Entgegenhaltungen:
- US-A- 4 774 020

## Beschreibung

Die vorliegende Erfindung betrifft neue 2-Phenylpyridine, deren Herstellung, flüssigkristalline Gemische, insbesondere Gemische mit getilteter oder chiral getilteter smektischer Phase, die solche Verbindungen enthalten, sowie die Verwendung für elektro-optische Zwecke.

Flüssige Kristalle eignen sich als Dielektrika in Anzeigenvorrichtungen, da ihre optischen Eigenschaften durch eine elektrische Spannung beeinflusst werden können. Geeignete elektro-optische Vorrichtungen sind dem Fachmann gut bekannt. Beispiele solcher Vorrichtungen sind Zellen mit dynamischer Streuung, DAP-Zellen (Deformation aufgerichteter Phasen), TN-Zellen (twisted-nematic) und STN-Zellen (super twisted-nematic) mit verdrillt nematischer Struktur, Gast/Wirt-Zellen, Phase-Change-Zellen mit einem cholesterisch-nematischen Phasenübergang und SBE-Zellen (super birefringence effect).

In Appl. Phys. Lett. 36, 899 (1980) und in Recent Developments in Condensed Matter Physics 4, 309 (1981) werden ferner elektro-optische Vorrichtungen auf der Basis von chiral getilteten smektischen Flüssigkristallen vorgeschlagen. Hierbei werden die ferroelektrischen Eigenschaften dieser Materialien ausgenutzt. Als getiltete smektische Phasen eignen sich beispielsweise smektisch C, F, G, H, I und K Phasen. Bevorzugt sind im allgemeinen smektisch C Phasen, welche besonders grosse Ansprechgeschwindigkeiten ermöglichen. Die chiral getilteten Phasen werden üblicherweise mit S_{C}*, S_{F}* etc. bezeichnet, wobei der Stern die Chiralität angibt.

Die Flüssigkristalle sollten eine gute Stabilität gegenüber chemischen und thermischen Einflüssen und gegenüber elektrischen und magnetischen Feldern aufweisen. Ferner sollten sie eine geeignete Mesophase über einen breiten Temperaturbereich, niedere Viskosität und kurze Ansprechzeiten besitzen. Ferroelektrische Flüssigkristalle sollten vorzugsweise eine breite chiral smektisch C Phase und eine ausreichend hohe spontane Polarisation besitzen. Um die Orientierung in der Zelle zu erleichtern, können sie ferner vorzugsweise eine smektisch A Phase oberhalb der smektisch C Phase aufweisen.

Flüssigkristalle werden in der Regel als Mischungen mehrerer Komponenten verwendet, um die verschiedenen Eigenschaften besser optimieren zu können. Neue Flüssigkristallkomponenten sollten daher vorzugsweise mit bekannten Materialien gut mischbar sein.

Als ferroelektrische Flüssigkristallmischungen eignen sich vorzugsweise Gemische bestehend aus einem oder mehreren optisch aktiven Dotierstoffen und einem Flüssigkristallmaterial (aus einer oder mehreren Komponenten), welches in der Regel eine breite getiltete smektische Phase, vorzugsweise eine smektisch C Phase aufweisen sollte. Die optisch aktiven Dotierstoffe müssen nicht selbst flüssigkristallin sein, können aber vorzugsweise eine smektische oder cholesterische Phase aufweisen. Die optisch aktiven Dotierstoffe sollten jedoch im Flüssigkristallmaterial eine chiral getiltete smektische Phase erzeugen und ferner eine ausreichend hohe spontane Polarisation und eine vergleichsweise kleine Verdrillung induzieren. Die Ganghöhe (Pitch) der Verdrillung sollte vorzugsweise deutlich grösser sein als der Plattenabstand der verwendeten Zelle und typischerweise mindestens etwa 10 µm betragen, um gut schaltende, bistabile Displays zu erhalten.

Gegenstand der vorliegenden Erfindung sind die Verbindungen der allgemeinen Formel 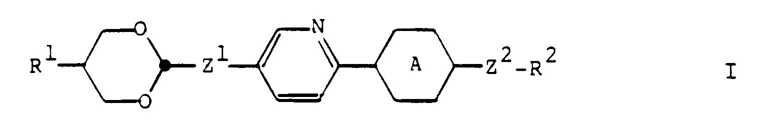
worin R¹ Alkyl oder Alkenyl bedeutet; Z¹ eine einfache Kovalenzbindung oder -CH₂CH₂- bezeichnet; Ring A unsubstituiertes oder mit Halogen und/oder Methyl substituiertes 1,4-Phenylen darstellt; Z² eine einfache Kovalenzbindung, Sauerstoff oder -CH₂O- bezeichnet; und R² eine unsubstituierte oder mit Halogen substituierte Alkyl- oder Alkenylgruppe bedeutet.

Die erfindungsgemässen Verbindungen sind farblos, besitzen eine hohe Stabilität und sind in üblichen Flüssigkristallmaterialien gut löslich. Sie besitzen eine ausgeprägte Tendenz zur Ausbildung einer smektisch C Phase in Flüssigkristallgemischen und weisen - insbesondere wenn Z² Sauerstoff bedeutet - meist selbst eine vergleichsweise breite smektisch C Phase auf. Ferner besitzen sie eine hohe Tendenz zur Ausbildung einer smektisch A Phase oberhalb der smektisch C Phase, wodurch die Ausrichtung des Flüssigkristalls in der Zelle erheblich erleichtert wird. Aufgrund dieser Eigenschaften sind auch Verbindungen, welche selbst keine oder nur eine enge smektisch C Phase aufweisen, sehr gut geeignet, die Eigenschaften von Gemischen zu verbessern. Weiterhin besitzen die erfindungsgemässen Verbindungen niedrige Viskositäten, insbesondere eine niedrige Rotationsviskosität, und ergeben somit in der Zelle kurze Schaltzeiten. Infolge der vorteilhaften Eigenschaften der Strukturen der Formel I können ferner durch Verwendung einer chiralen Gruppe in R¹ und/oder R² chirale Dotierstoffe erhalten werden, welche mit den Flüssigkristallmaterialien sehr gut kompatibel sind und häufig selbst eine chiral getiltete smektische Phase aufweisen.

Der Ausdruck "Halogen" umfasst im Rahmen der vorliegenden Erfindung Fluor, Chlor, Brom und Jod, insbesondere Fluor und Chlor.

Der Ausdruck "unsubstituiertes oder mit Halogen und/oder Methyl substituiertes 1,4-Phenylen" umfasst 1,4-Phenylen, 2-Fluor-1,4-phenylen, 2-Chlor-1,4-phenylen, 2-Brom-1,4-phenylen, 2-Jod-1,4-phenylen, 2-Methyl-1,4-phenylen, 2,3-Difluor-1,4-phenylen und dergleichen.

Der Ausdruck "Alkyl" umfasst geradkettige und verzweigte Reste wie Methyl, Aethyl, Propyl, Butyl, Pentyl, Hexyl, Heptyl, Octyl, Nonyl, Decyl, Undecyl, Dodecyl, 1-Methylheptyl (= 2-Octyl), 3-Methylpentyl, 4-Methylhexyl, 5-Methylheptyl, 6-Methyloctyl und dergleichen. Alkylreste mit 1-16 Kohlenstoffatomen sind bevorzugt.

Der Ausdruck "Alkenyl" umfasst geradkettige und verzweigte Reste wie Vinyl, Allyl, Propenyl, Butenyl, Pentenyl, Hexenyl, Heptenyl, Octenyl, Nonenyl, Decenyl, Undecenyl, Dodecenyl, 3,7-Dimethyl-6-octenyl und dergleichen. Alkenylreste mit 2-16 Kohlenstoffatomen sind bevorzugt. Insbesondere steht der Ausdruck "Alkenyl" (in R¹ und/oder R²) für Gruppen mit endständiger Doppelbindung wie Vinyl, Allyl, 3-Butenyl, 4-Pentenyl, 5-Hexenyl, 6-Heptenyl, 7-Octenyl, 8-Nonenyl, 9-Decenyl, 10-Undecenyl, 11-Dodecenyl und dergleichen. In R¹ steht ferner der Ausdruck "Alkenyl" insbesondere für 1E-Alkenyl, wie Vinyl, 1E-Propenyl, 1E-Butenyl, 1E-Pentenyl, 1E-Hexenyl, 1E-Heptenyl, 1E-Octenyl, 1E-Nonenyl, 1E-Decenyl, 1E-Undecenyl und 1E-Dodecenyl, für 3E-Alkenyl, wie 3-Butenyl, 3E-Pentenyl, 3E-Hexenyl, 3E-Heptenyl, 3E-Octenyl, 3E-Nonenyl, 3E-Decenyl, 3E-Undecenyl und 3E-Dodecenyl, und für 4-Alkenyl (bevorzugt 4Z-Alkenyl), wie 4-Pentenyl, 4-Hexenyl, 4-Heptenyl, 4-Octenyl, 4-Nonenyl, 4-Decenyl, 4-Undecenyl und 4-Dodecenyl. Wenn Z² eine einfache Kovalenzbindung bezeichnet, steht der Ausdruck "Alkenyl" in R² neben den Gruppen mit endständiger Doppelbindung insbesondere auch für 3E-Alkenyl und für 4-Alkenyl, beispielsweise für die unter R¹ genannten 3E-Alkenyl- und 4-Alkenylgruppen. Wenn Z² Sauerstoff bezeichnet, steht der Ausdruck "Alkenyl" in R² neben den Gruppen mit endständiger Doppelbindung insbesondere auch für 2E-Alkenyl, wie Allyl, 2E-Butenyl, 2E-Pentenyl, 2E-Hexenyl, 2E-Heptenyl, 2E-Octenyl, 2E-Nonenyl, 2E-Decenyl, 2E-Undecenyl, 2E-Dodecenyl, und für 3-Alkenyl (bevorzugt 3Z-Alkenyl) , wie 3-Butenyl, 3-Pentenyl, 3-Hexenyl, 3-Heptenyl, 3-Octenyl, 3-Nonenyl, 3-Decenyl, 3-Undecenyl und 3-Dodecenyl. Wenn Z² -CH₂O- bezeichnet, steht der Ausdruck "Alkenyl" in R² neben den Gruppen mit endständiger Doppelbindung insbesondere auch für 2-Alkenyl (bevorzugt 2Z-Alkenyl) , wie Allyl, 2-Butenyl, 2-Pentenyl, 2-Hexenyl, 2-Heptenyl, 2-Octenyl, 2-Nonenyl, 2-Decenyl, 2-Undecenyl und 2-Dodecenyl.

Der Ausdruck "unsubstituierte oder mit Halogen substituierte Alkyl- oder Alkenylgruppe" umfasst die obengenannten Alkylgruppen und Alkenylgruppen sowie davon abgeleitete Haloalkyl- oder Haloalkenylgruppen, wie 1-Fluoralkyl, 1-Chloralkyl, 2-Fluoralkyl, 2-Chloralkyl und dergleichen.

Im allgemeinen sind diejenigen Verbindungen der Formel I bevorzugt, worin Ring A unsubstituiertes, monosubstituiertes oder 2,3-disubstituiertes 1,4-Phenylen (insbesondere 1,4-Phenylen, 2-Fluor-1,4-phenylen oder 2,3-Difluor-1,4-phenylen) darstellt. Bevorzugte erfindungsgemässe Verbindungen sind somit die Verbindungen der allgemeinen Formeln 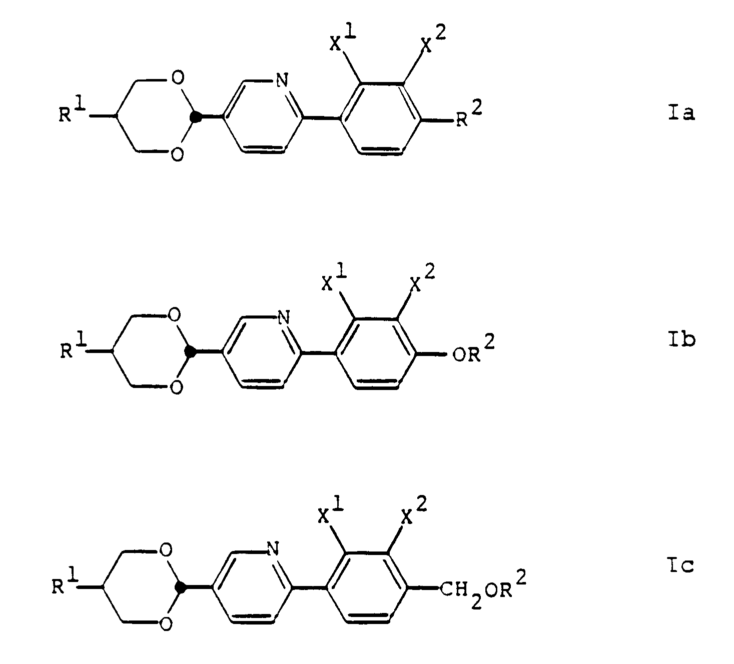 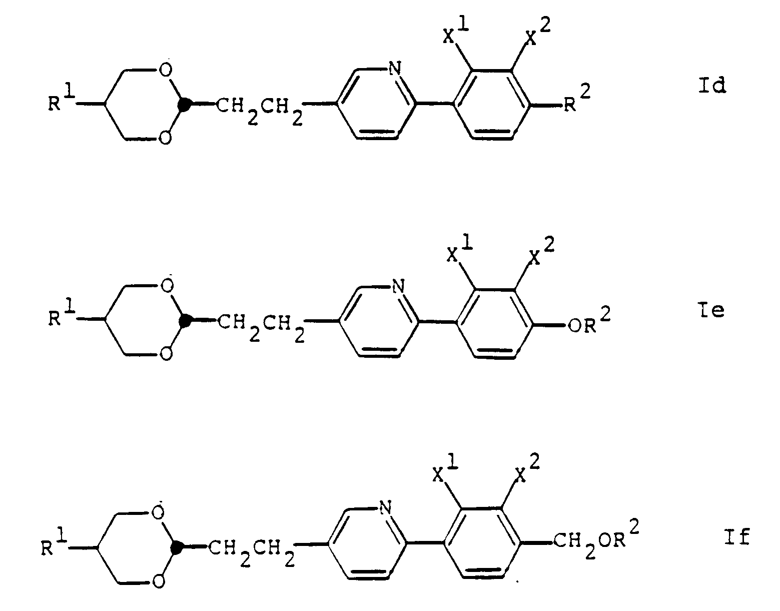
worin R¹ und R² die obigen Bedeutungen haben und X¹ und X² unabhängig voneinander Wasserstoff, Halogen oder Methyl bezeichnen.

Besonders bevorzugt sind diejenigen Verbindungen der Formeln Ia - If, worin X¹ und X² unabhängig voneinander jeweils Wasserstoff oder Fluor bedeuten, insbesondere diejenigen, worin X¹ Wasserstoff und X² Wasserstoff oder Fluor bedeuten.

Rest R¹ in den obigen Formeln I und Ia - If besitzt vorzugsweise etwa 3 - 14 Kohlenstoffatome, besonders bevorzugt etwa 7 - 12 Kohlenstoffatome. Bevorzugte Reste R² in den obigen Formeln I und Ia - If sind im allgemeinen diejenigen mit etwa 3 - 12 Kohlenstoffatomen, insbesondere diejenigen mit etwa 6 - 10 Kohlenstoffatomen. Kürzere und längere Ketten sind für den vorliegenden Zweck ebenfalls geeignet, aber kürzere Ketten können engere Mesophasebereiche der reinen Verbindung ergeben und längere Ketten ergeben meist keine oder nur noch eine unwesentliche Verbesserung gegenüber den genannten Kettenlängen.

Bevorzugte Reste R¹ in den obigen Formeln I und Ia - If sind 1E-Alkenyl, 3E-Alkenyl, Alkenylgruppen mit einer endständigen Doppelbindung und insbesondere Alkyl. Geradkettige Reste R¹ sind im allgemeinen bevorzugt.

Bevorzugte Reste R² in den Formeln Ia und Id - bzw. in Formeln I, wenn Z² eine einfache Kovalenzbindung bezeichnet, - sind 3E-Alkenyl, Alkenyl mit endständiger Doppelbindung und insbesondere Alkyl, sowie entsprechende Haloalkenyl- und Haloalkylgruppen, insbesondere 1-Fluoralkyl, 1-Chloralkyl, 2-Fluoralkyl und 2-Chloralkyl.

Bevorzugte Reste R² in den Formeln Ib und Ie - bzw. in Formeln I, wenn Z² Sauerstoff bedeutet, - sind 2E-Alkenyl und insbesondere Alkenyl mit endständiger Doppelbindung und Alkyl, sowie entsprechende Haloalkenyl- und Haloalkylgruppen, insbesondere 2-Fluoralkyl und 2-Chloralkyl.

Bevorzugte Reste R² in den Formeln Ic und If - bzw. in Formeln I, wenn Z² -CH₂O- bezeichnet, - sind Alkenyl mit endständiger Doppelbindung und Alkyl, sowie entsprechende Haloalkenyl- und Haloalkylgruppen, insbesondere 2-Fluoralkyl und 2-Chloralkyl.

Die Alkyl- und Alkenylgruppen in R² sind vorzugsweise geradkettig oder können - insbesondere wenn eine optisch aktive Verbindung gewünscht ist - vorzugsweise auch eine Methylverzweigung aufweisen. Bevorzugte verzweigte Gruppen sind die Methylalkylgruppen, insbesondere 1-Methylalkyl (z.B. 1-Methylheptyl) und Alkylreste, welche die Gruppierung -CH(CH₃)CH₂CH₃ aufweisen (z.B. 4-Methylhexyl, 5-Methylheptyl, 6-Methyloctyl).

Die Verbindungen der Formel I können erfindungsgemäss dadurch hergestellt werden, dass man einen Aldehyd der allgemeinen Formel 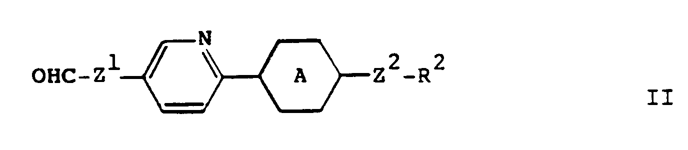
oder ein Acetal hiervon mit einer Verbindung der allgemeinen Formel 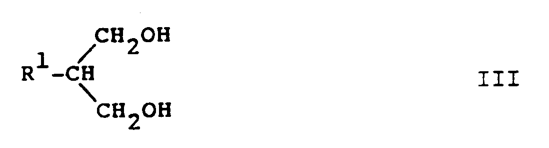
worin R¹, R², Z¹, Z² und Ring A die obigen Bedeutungen haben,
umsetzt.

Die Umsetzung des Aldehyds oder eines geeigneten Acetals (z.B. des Dimethylacetals) mit dem Diol der Formel III kann in an sich bekannter Weise erfolgen.

Zweckmässigerweise erfolgt die Umsetzung in einem inerten organischen Lösungsmittel (beispielsweise einem aromatischen Kohlenwasserstoff, wie Benzol, Toluol oder Xylol) in Gegenwart einer katalytischen Menge einer organischen oder anorganischen Säure, wie p-Toluolsulfonsäure, Schwefelsäure oder trockenem Chlorwasserstoff. Temperatur und Druck sind nicht kritisch, vorzugsweise wird jedoch bei Rückflusstemperatur (unter Abtrennung des gebildeten Wassers) und Atmosphärendruck gearbeitet.

Verbindungen der Formel I, worin Z² Sauerstoff oder -CH₂O- bezeichnet und R² Alkenyl oder eine mit Halogen substitutierte Gruppe bedeutet, können vorzugsweise auch via Verbindungen der Formel I oder II, worin R² Alkyl bedeutet, erhalten werden durch Dealkylierung und anschliessende Verätherung mit der gewünschten Gruppe. Diese Umätherung kann vor oder nach der Verknüpfung zum Dioxan erfolgen. Geeignete Methoden sind dem Fachmann bekannt, z.B. aus Synthesis 1988, 749 und der dort zitierten Literatur.

Die Ausgangsmaterialien der Formel III sind bekannte oder Analoge bekannter Verbindungen. Geeignete Methoden zur Herstellung dieser Verbindungen sind dem Fachmann bekannt, z.B. aus EP-A-0122389, EP-A-0169327, EP-A-0167912 und EP-A-0168683.

Die Herstellung der Aldehyde der Formel II wird anhand der folgenden Reaktionsschemata illustriert, worin THPO Tetrahydro-2-pyranyloxy bezeichnet und R², Z² und Ring A die obigen Bedeutungen haben: 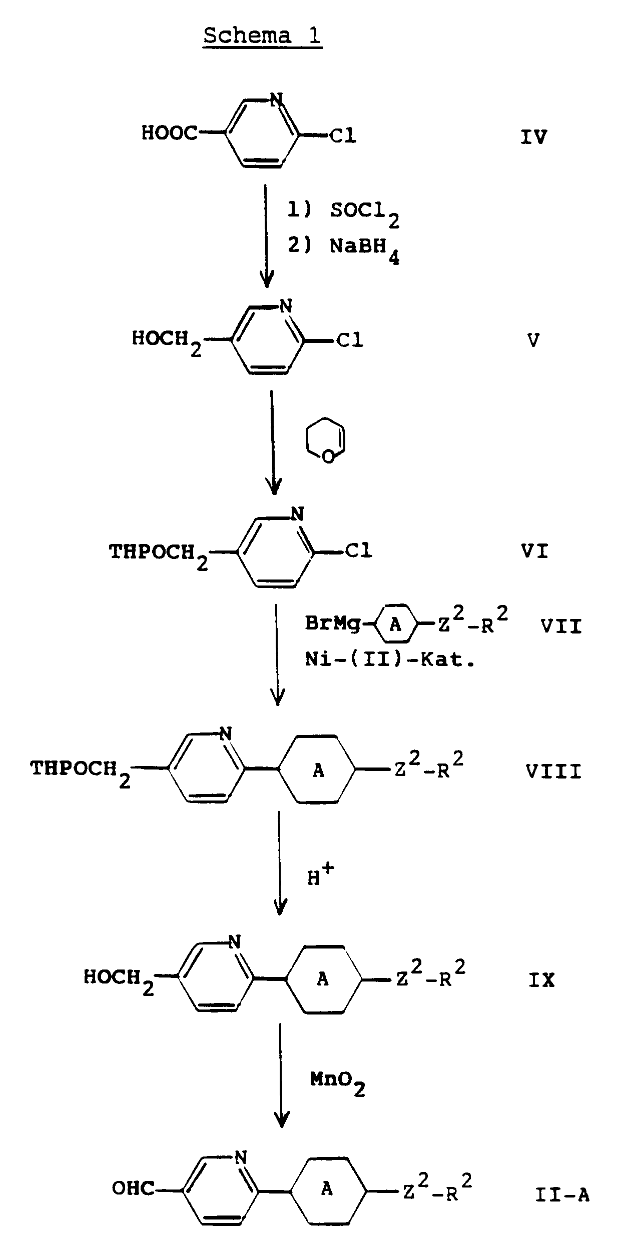 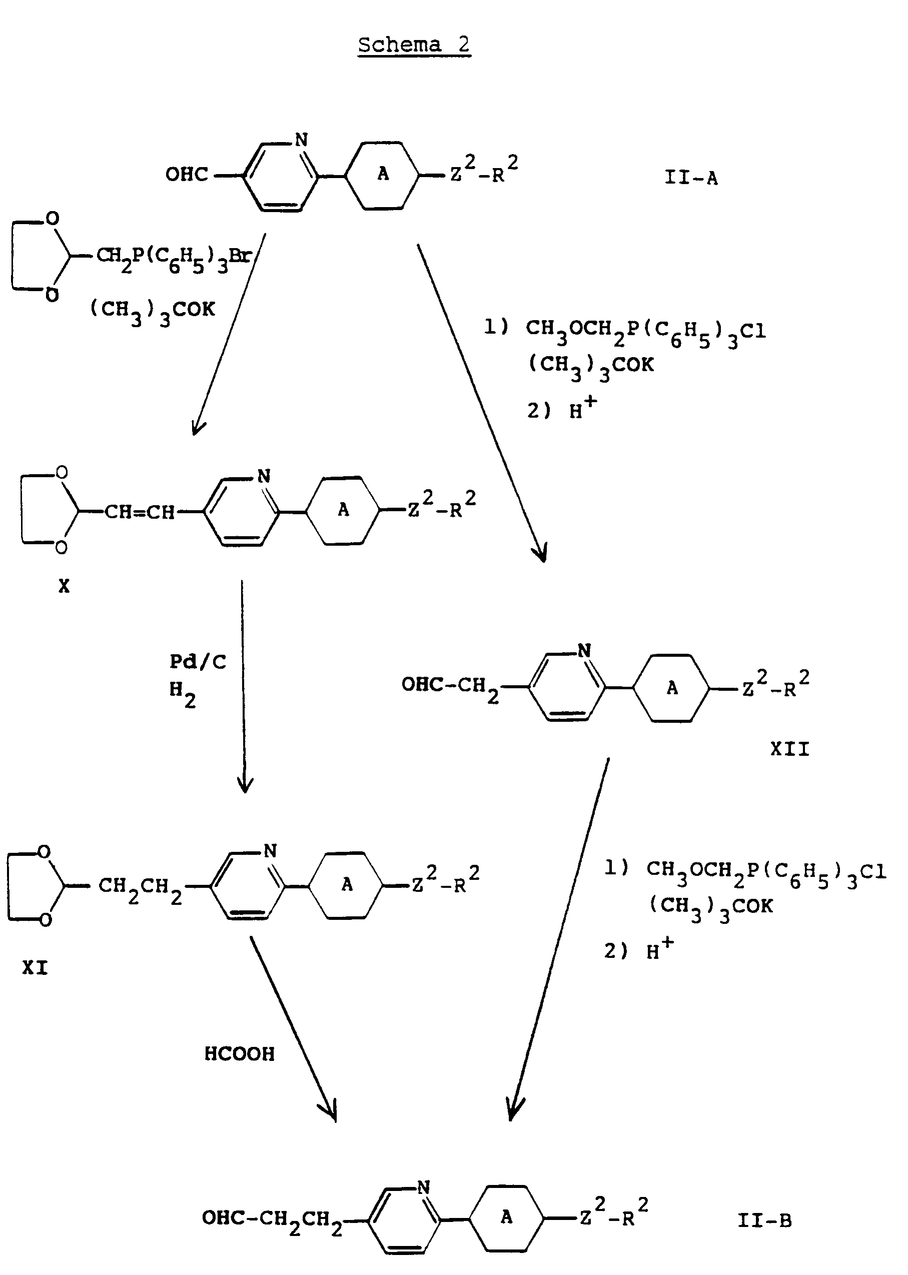
Die Ausgangsmaterialien der Formeln IV und V sind bekannt. Die Grignard-Verbindungen der Formel VII bzw. die entsprechenden Brombenzole sind bekannte oder Analoge bekannter Verbindungen. Ein geeigneter Nickel-Katalysator zur Umsetzung der Verbindung der Formel VI ist beispielsweise 1,3-Bis(diphenylphosphino)propannickel-(II)-chlorid. Wenn R² eine Doppelbindung oder einen Halogensubstituenten aufweist, erfolgt die Kettenverlängerung nach Schema 2 zweckmässig via Verbindung der Formel XII, um eine allfällige Reduktion zu vermeiden.

Die erfindungsgemässen Verbindungen können in Form von Gemischen untereinander und/oder mit andern Flüssigkristallkomponenten verwendet werden. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Die Erfindung betrifft daher ebenfalls ein flüssigkristallines Gemisch mit mindestens 2 Komponenten, welches dadurch gekennzeichnet ist, dass mindestens eine Komponente eine Verbindung der Formel I (insbesondere eine der als bevorzugt genannten Verbindungen) ist. Die Verbindungen der Formel I sind auch für nematische oder cholesterische Gemische geeignet. Bevorzugt sind jedoch Gemische mit getilteter smektischer Phase, insbesondere Gemische mit chiral getilteter smektischer Phase. Die getiltete oder chiral getiltete Phase ist vorzugsweise eine smektisch C Phase. Die chiral getilteten smektischen Gemische können vorzugsweise aus einem oder mehreren optisch aktiven Dotierstoffen und einem Flüssigkristallmaterial (bestehend aus einer oder mehreren Komponenten) mit getilteter smektischer Phase bestehen und sind dadurch gekennzeichnet, dass mindestens eine Komponente des Flüssigkristallmaterials eine Verbindung der Formel I ist.

Aufgrund der vorteilhaften Eigenschaften kann der Anteil an Verbindungen der Formel I in den erfindungsgemässen Gemischen in einem breiten Bereich variieren und beispielsweise etwa 0,5 - 60 Gew.-% oder mehr betragen. Bevorzugt ist im allgemeinen ein Anteil von etwa 3 - 40 Gew.-%, insbesondere etwa 10 - 30 Gew.-% an Verbindungen der Formel I. Der Anteil an chiralen Dotierstoffen der Formel I kann jedoch vorzugsweise auch geringer sein und beispielsweise etwa 0,5 - 10 Gew.-% betragen.

Die erfindungsgemässen Gemische für smektische Anwendungen (insbesondere für getiltete smektische oder chiral getiltete smektische Phasen) enthalten neben einer oder mehreren Verbindungen der Formel I vorzugsweise eine oder mehrere Verbindungen aus der Gruppe der Verbindungen der allgemeinen Formeln 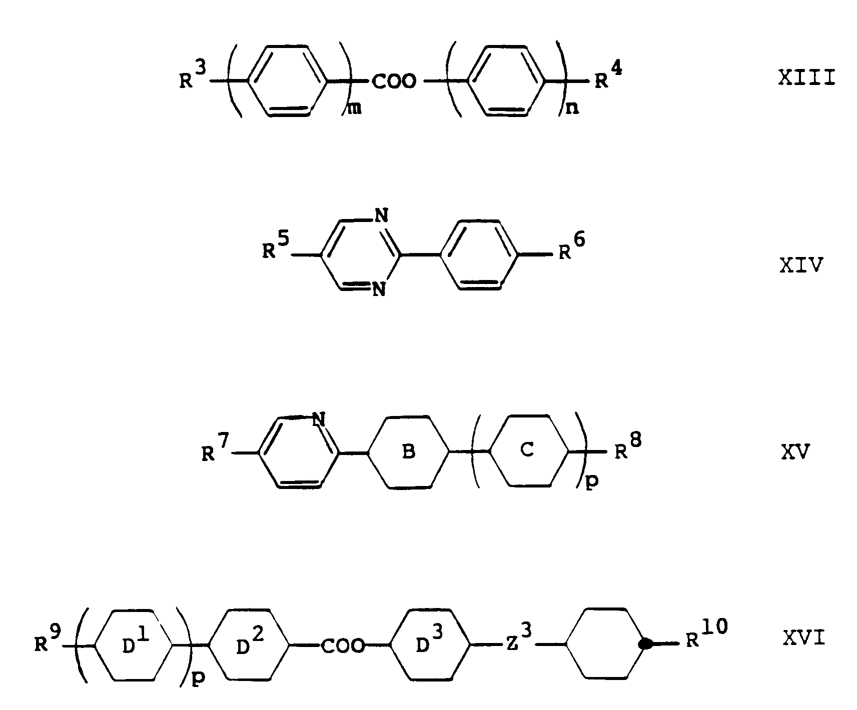 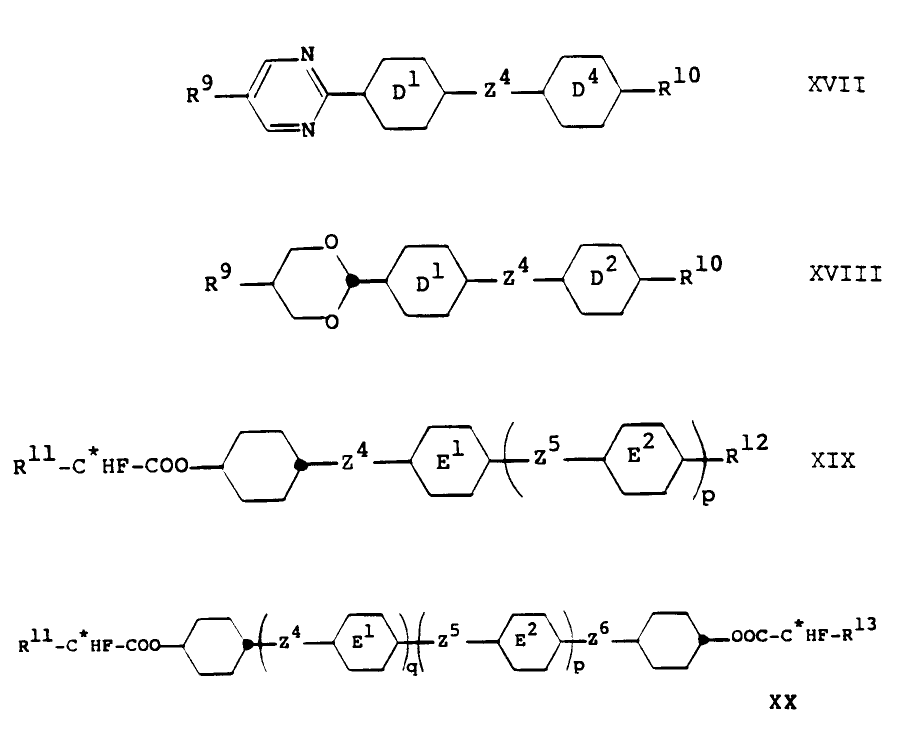
worin R³ und R⁴ Alkyl, Alkoxy, Alkenyloxy, Alkanoyloxy oder Alkoxycarbonyl mit bis zu 18 Kohlenstoffatomen bezeichnen; m und n unabhängig voneinander 1 oder 2 bedeuten; R⁵ und R⁶ Alkyl, Alkoxy oder Alkenyloxy mit bis zu 18 Kohlenstoffatomen bezeichnen; Ring B unsubstituiertes oder mit Halogen und/oder Methyl substituiertes 1,4-Phenylen darstellt; Ring C trans-1,4-Cyclohexylen oder unsubstituiertes oder mit Halogen und/oder Methyl substituiertes 1,4-Phenylen darstellt; p und q unabhängig voneinander für die Zahl 0 oder 1 stehen; R⁷ und R⁸ unabhängig voneinander eine unsubstituierte oder mit Halogen substituierte C₁-C₁₈-Alkyl- oder C₂-C₁₈-Alkenylgruppe bezeichnen, in welcher gegebenenfalls eine oder zwei nicht benachbarte CH₂-Gruppen durch -O-, -COO- und/oder -OOC-ersetzt sind; die Ringe D¹, D² und D³ unabhängig voneinander unsubstituiertes oder mit Cyano, Halogen oder Niederalkyl substitutiertes 1,4-Phenylen darstellen; Z³ eine einfache Kovalenzbindung, -CH₂CH₂-, -OCH₂-, -COO- oder -OOC- bezeichnet; R⁹ und R¹⁰ unabhängig voneinander eine unsubstituierte oder halogensubstituierte C₁-C₁₈-Alkyl- oder C₂-C₁₈-Alkenylgruppe bedeuten, in welcher gegebenenfalls eine oder zwei nicht benachbarte CH₂-Gruppen durch Sauerstoff ersetzt sind; Ring D⁴ trans-1,4-Cyclohexylen oder unsubstituiertes oder mit Cyano, Halogen oder Niederalkyl substituiertes 1,4-Phenylen darstellt; Z⁴, Z⁵ und Z⁶ unabhängig voneinander eine einfache Kovalenzverbindung, -COO-, -OOC-, -CH₂CH₂-, -OCH₂- oder -CH₂O- bezeichnen; R¹¹ und R¹³ unabhängig voneinander eine C₁-C₁₅-Alkyl- oder C₂-C₁₅-Alkenylgruppe bedeuten, in welcher gegebenenfalls eine CH₂-Gruppe durch Sauerstoff ersetzt ist; R¹² eine unsubstituierte oder mit Halogen substituierte C₁-C₁₅-Alkyl- oder C₂-C₁₅-Alkenylgruppe bezeichnet, in welcher gegebenenfalls eine CH₂-Gruppe durch Sauerstoff ersetzt ist und/oder gegebenenfalls eine CH₂-Gruppe durch eine Estergruppe -COO- oder -OOC- ersetzt ist; die Ringe E¹ und E² unabhängig voneinander unsubstituiertes oder mit Halogen, Cyano und/oder Methyl substituiertes 1,4-Phenylen darstellen, in welchem gegebenenfalls 1 oder 2 CH-Gruppen durch Stickstoff ersetzt sind; und C* ein chirales Kohlenstoffatom bezeichnet.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. Die optischen Antipoden von chiralen Verbindungen besitzen jeweils die gleichen Phasenumwandlungstemperaturen und absolut gleiche Werte der Verdrillung aber mit entgegengesetzten Vorzeichen. Die zur Charakterisierung der Phasenumwandlungen verwendeten Abkürzungen stehen für folgende Bedeutungen:
- C: für kristallin
- S: für smektisch
- S_{A}, S_{B}, S_{C}: etc. für smektisch A, B, C etc.
- S_{C}*, S_{F}*: für chiral smektisch C, F etc.
- N: für nematisch
- N*: für cholesterich
- I: für isotrop.

### Beispiel 1

Eine Lösung von 2,2 g 6-[4-(Octyloxy)phenyl]-3-pyridincarboxaldehyd (hergestellt gemäss Beispiel 2) und 1,4 g 2-Decyl-1,3-propandiol in 40 ml Toluol wurde mit 5 Tropfen 10-prozentiger (v/v) Schwefelsäure versetzt. Das Gemisch wurde 1 Stunde zum Sieden erhitzt, wobei feuchtes Toluol abdestilliert und durch frisches Toluol ersetzt wurde. Dann wurde das Reaktionsgemisch mit Triäthylamin neutralisiert und nach dem Erkalten dreimal mit Wasser gewaschen, über Natriumsulfat getrocknet und eingeengt. Chromatographische Reinigung des Rückstandes an 60 g Kieselgel mit Hexan/Aethylacetat (Vol. 9:1) und mehrmalige Umkristallisation des trans/cis-Gemisches aus Aethylacetat ergab 0,56 g reines 5-(trans-5-Decyl-1,3-dioxan-2-yl)-2-[4-(octyloxy)phenyl]pyridin; Smp. (C-S) 69,7°C, Uebergang S-S_{C} 137,5°C, Klp. (S_{C}-I) 179°C.

In analoger Weise können folgende Verbindungen hergestellt werden:
5-(trans-5-Octyl-1,3-dioxan-2-yl)-2-[4-(hexyloxy)phenyl]pyridin;
5-(trans-5-Nonyl-1,3-dioxan-2-yl)-2-[4-(hexyloxy)phenyl]pyridin;
5-(trans-5-Decyl-1,3-dioxan-2-yl)-2-[4-(hexyloxy)phenyl]pyridin;
5-(trans-5-Dodecyl-1,3-dioxan-2-yl)-2-[4-(hexyloxy)phenyl]pyridin;
5-(trans-5-Heptyl-1,3-dioxan-2-yl)-2-[4-(heptyloxy)phenyl]pyridin;
5-(trans-5-Octyl-1,3-dioxan-2-yl)-2-[4-(heptyloxy)phenyl]pyridin;
5-(trans-5-Nonyl-1,3-dioxan-2-yl)-2-[4-(heptyloxy)phenyl]pyridin;
5-(trans-5-Decyl-1,3-dioxan-2-yl)-2-[4-(heptyloxy)phenyl]pyridin;
5-(trans-5-Dodecyl-1,3-dioxan-2-yl)-2-[4-(heptyloxy)phenyl]pyridin;
5-(trans-5-Hexyl-1,3-dioxan-2-yl)-2-[4-(octyloxy)phenyl]pyridin;
5-(trans-5-Heptyl-1,3-dioxan-2-yl)-2-[4-(octyloxy)phenyl]pyridin, Smp. (C-S) 76,1°C, S-S_{C} 137,5°C, S_{C}-S_{A} 169,5°C, S_{A}-N 181,5°C, Klp. (N-I) 187°C;
5-(trans-5-Octyl-1,3-dioxan-2-yl)-2-[4-(octyloxy)phenyl]pyridin, Smp. (C-S) 67,3°C, S-S_{C} 139,5°C, S_{C}-S_{A} 177°C, S_{A}-N 182°C, Klp. (N-I) 185°C;
5-(trans-5-Nonyl-1,3-dioxan-2-yl)-2-[4-(octyloxy)phenyl]pyridin;
5-(trans-5-Dodecyl-1,3-dioxan-2-yl)-2-[4-(octyloxy)phenyl]pyridin;
5-(trans-5-Heptyl-1,3-dioxan-2-yl)-2-[4-(nonyloxy)phenyl]pyridin;
5-(trans-5-Octyl-1,3-dioxan-2-yl)-2-[4-(nonyloxy)phenyl]pyridin;
5-(trans-5-Decyl-1,3-dioxan-2-yl)-2-[4-(nonyloxy)phenyl]pyridin;
5-(trans-5-Hexyl-1,3-dioxan-2-yl)-2-[4-(decyloxy)phenyl]pyridin;
5-(trans-5-Heptyl-1,3-dioxan-2-yl)-2-[4-(decyloxy)phenyl]pyridin;
5-(trans-5-Octyl-1,3-dioxan-2-yl)-2-[4-(decyloxy)phenyl]pyridin;
5-(trans-5-Nonyl-1,3-dioxan-2-yl)-2-[4-(decyloxy)phenyl]pyridin;
5-(trans-5-Decyl-1,3-dioxan-2-yl)-2-[4-(decyloxy)phenyl]pyridin;
5-(trans-5-[10-Undecenyl]-1,3-dioxan-2-yl)-2-[4-(hexyloxy)phenyl]pyridin;
5-(trans-5-[10-Undecenyl]-1,3-dioxan-2-yl)-2-[4-(octyloxy)phenyl]pyridin;
5-(trans-5-[10-Undecenyl]-1,3-dioxan-2-yl)-2-[4-(decyloxy)phenyl]pyridin;
5-(trans-5-Octyl-1,3-dioxan-2-yl)-2-[4-[(S)-4-methylhexyloxy]phenyl]pyridin;
5-(trans-5-Decyl-1,3-dioxan-2-yl)-2-[4-[(S)-4-methylhexyloxy]phenyl]pyridin;
5-(trans-5-Octyl-1,3-dioxan-2-yl)-2-[4-[(S)-5-methylheptyloxy]phenyl]pyridin;
5-(trans-5-Decyl-1,3-dioxan-2-yl)-2-[4-[(S)-5-methylheptyloxy]phenyl]pyridin;
5-(trans-5-Octyl-1,3-dioxan-2-yl)-2-[4-[(S)-6-methyloctyloxy]phenyl]pyridin;
5-(trans-5-Decyl-1,3-dioxan-2-yl)-2-[4-[(S)-6-methyloctyloxy]phenyl]pyridin;
5-(trans-5-Octyl-1,3-dioxan-2-yl)-2-[4-[(R)-2-fluoroctyloxy)phenyl]pyridin;
5-(trans-5-Octyl-1,3-dioxan-2-yl)-2-[4-[(S)-2-fluoroctyloxy]phenyl]pyridin;
5-(trans-5-Heptyl-1,3-dioxan-2-yl)-2-[3-fluor-4-(hexyloxy)phenyl]pyridin;
5-(trans-5-Octyl-1,3-dioxan-2-yl)-2-[3-fluor-4-(hexyloxy)phenyl]pyridin, Smp. (C-S_{C}) 72,7°C, S_{C}-S_{A} 146°C, Klp. (S_{A}-I) 174,5°C;
5-(trans-5-Nonyl-1,3-dioxan-2-yl)-2-[3-fluor-4-(hexyloxy)phenyl]pyridin;
5-(trans-5-Decyl-1,3-dioxan-2-yl)-2-[3-fluor-4-(hexyloxy)phenyl]pyridin, Smp. (C-S_{C}) 76,5°C, S_{C}-S_{A} 159°C, Klp. (S_{A}-I) 172,3°C;
5-(trans-5-Dodecyl-1,3-dioxan-2-yl)-2-[3-fluor-4-(hexyloxy)phenyl]pyridin, Smp. (C-S_{C}) 79,1°C, S_{C}-S_{A} 160°C, Klp. (S_{A}-I) 168,5°C;
5-(trans-5-Heptyl-1,3-dioxan-2-yl)-2-[3-fluor-4-(heptyloxy)phenyl]pyridin;
5-(trans-5-Octyl-1,3-dioxan-2-yl)-2-[3-fluor-4-(heptyloxy)phenyl]pyridin, Smp. (C-S_{C}) 80,6°C, S_{C}-S_{A} 145°C, Klp (S_{A}-I) 171°C;
5-(trans-5-Nonyl-1,3-dioxan-2-yl)-2-[3-fluor-4-(heptyloxy)phenyl]pyridin;
5-(trans-5-Decyl-1,3-dioxan-2-yl)-2-[3-fluor-4-(heptyloxy)phenyl]pyridin, Smp. (C-S_{C}) 82,3°C, S_{C}-S_{A} 160°C, Klp. (S_{A}-I) 169°C;
5-(trans-5-Dodecyl-1,3-dioxan-2-yl)-2-[3-fluor-4-(heptyloxy)phenyl]pyridin, Smp. (C-S_{C}) 86,5°C, S_{C}-S_{A} 161,5°C, Klp. (S_{A}-I) 165°C;
5-(trans-5-Heptyl-1,3-dioxan-2-yl)-2-[3-fluor-4-(octyloxy)phenyl]pyridin, Smp. (C-S_{C}) 80,6°C, S_{C}-S_{A} 111°C, Klp. (S_{A}-I) 174,5°C;
5-(trans-5-Octyl-1,3-dioxan-2-yl)-2-[3-fluor-4-(octyloxy)phenyl]pyridin;
5-(trans-5-Nonyl-1,3-dioxan-2-yl)-2-[3-fluor-4-(octyloxy)phenyl]pyridin;
5-(trans-5-Decyl-1,3-dioxan-2-yl)-2-[3-fluor-4-(octyloxy)phenyl]pyridin, Smp. (C-S_{C}) 77,8°C, S_{C}-S_{A} 160,5°C, Klp. (S_{A}-I) 167,5°C;
5-(trans-5-Dodecyl-1,3-dioxan-2-yl)-2-[3-fluor-4-(octyloxy)phenyl]pyridin;
5-(trans-5-Octyl-1,3-dioxan-2-yl)-2-[3-fluor-4-(decyloxy)phenyl]pyridin, Smp. (C-S_{C}) 81,4°C, S_{C}-S_{A} 136°C, Klp. (S_{A}-I) 167,5°C;
5-(trans-5-Nonyl-1,3-dioxan-2-yl)-2-[3-fluor-4-(decyloxy)phenyl]pyridin;
5-(trans-5-Decyl-1,3-dioxan-2-yl)-2-[3-fluor-4-(decyloxy)phenyl]pyridin, Smp. (C-S_{C}) 81,1°C, S_{C}-S_{A} 158°C, Klp. (S_{A}-I) 163,5°C;
5-(trans-5-Dodecyl-1,3-dioxan-2-yl)-2-[3-fluor-4-(decyloxy)phenyl]pyridin, Smp. (C-S_{C}) 84,8°C, S_{C}-S_{A} 159,5°C, Klp. (S_{A}-I) 159,7°C;
5-(trans-5-Decyl-1,3-dioxan-2-yl)-2-[2-fluor-4-(hexyloxy)phenyl]pyridin;
5-(trans-5-Octyl-1,3-dioxan-2-yl)-2-[2-fluor-4-(octyloxy)phenyl]pyridin;
5-(trans-5-Decyl-1,3-dioxan-2-yl)-2-[2-fluor-4-(octyloxy)phenyl]pyridin;
5-(trans-5-Dodecyl-1,3-dioxan-2-yl)-2-[2-fluor-4-(octyloxy)phenyl]pyridin;
5-(trans-5-Octyl-1,3-dioxan-2-yl)-2-[2-fluor-4-(decyloxy)phenyl]pyridin;
5-(trans-5-Decyl-1,3-dioxan-2-yl)-2-[2-fluor-4-(decyloxy)phenyl]pyridin;
5-(trans-5-Octyl-1,3-dioxan-2-yl)-2-[2,3-difluor-4-(hexyloxy)phenyl]pyridin;
5-(trans-5-Decyl-1,3-dioxan-2-yl)-2-[2,3-difluor-4-(hexyloxy)phenyl]pyridin;
5-(trans-5-Dodecyl-1,3-dioxan-2-yl)-2-[2,3-difluor-4-(hexyloxy)phenyl]pyridin;
5-(trans-5-Octyl-1,3-dioxan-2-yl)-2-[2,3-difluor-4-(heptyloxy)phenyl]pyridin;
5-(trans-5-Decyl-1,3-dioxan-2-yl)-2-[2,3-difluor-4-(heptyloxy)phenyl]pyridin;
5-(trans-5-Dodecyl-1,3-dioxan-2-yl)-2-[2,3-difluor-4-(heptyloxy)phenyl]pyridin;
5-(trans-5-Octyl-1,3-dioxan-2-yl)-2-[2,3-difluor-4-(octyloxy)phenyl]pyridin;
5-(trans-5-Nonyl-1,3-dioxan-2-yl)-2-[2,3-difluor-4-(octyloxy)phenyl]pyridin;
5-(trans-5-Decyl-1,3-dioxan-2-yl)-2-[2,3-difluor-4-(octyloxy)phenyl]pyridin;
5-(trans-5-Dodecyl-1,3-dioxan-2-yl)-2-[2,3-difluor-4-(octyloxy)phenyl]pyridin;
5-(trans-5-Decyl-1,3-dioxan-2-yl)-2-[2,3-difluor-4-(nonyloxy)phenyl]pyridin;
5-(trans-5-Octyl-1,3-dioxan-2-yl)-2-[2,3-difluor-4-(decyloxy)phenyl]pyridin;
5-(trans-5-Decyl-1,3-dioxan-2-yl)-2-[2,3-difluor-4-(decyloxy)phenyl]pyridin;
5-(trans-5-Dodecyl-1,3-dioxan-2-yl)-2-[2,3-difluor-4-(decyloxy)phenyl]pyridin;
5-(trans-5-Decyl-1,3-dioxan-2-yl)-2-[3-chlor-4-(hexyloxy)phenyl]pyridin;
5-(trans-5-Octyl-1,3-dioxan-2-yl)-2-[3-chlor-4-octyloxy)phenyl]pyridin;
5-(trans-5-Decyl-1,3-dioxan-2-yl)-2-[3-chlor-4-(octyloxy)phenyl]pyridin;
5-(trans-5-Dodecyl-1,3-dioxan-2-yl)-2-[3-chlor-4-(octyloxy)phenyl]pyridin;
5-(trans-5-Octyl-1,3-dioxan-2-yl)-2-[3-chlor-4-(decyloxy)phenyl]pyridin;
5-(trans-5-Decyl-1,3-dioxan-2-yl)-2-[3-chlor-4-(decyloxy)phenyl]pyridin;
5-(trans-5-Decyl-1,3-dioxan-2-yl)-2-[3-methyl-4-(hexyloxy)phenyl]pyridin;
5-(trans-5-Octyl-1,3-dioxan-2-yl)-2-[3-methyl-4-(octyloxy)phenyl]pyridin;
5-(trans-5-Decyl-1,3-dioxan-2-yl)-2-[3-methyl-4-(octyloxy)phenyl]pyridin;
5-(trans-5-Dodecyl-1,3-dioxan-2-yl)-2-[3-methyl-4-octyloxy)phenyl]pyridin;
5-(trans-5-Octyl-1,3-dioxan-2-yl)-2-[3-methyl-4-(decyloxy)phenyl]pyridin;
5-(trans-5-Decyl-1,3-dioxan-2-yl)-2-[3-methyl-4-(decyloxy)phenyl]pyridin;
5-(trans-5-Decyl-1,3-dioxan-2-yl)-2-[4-heptylphenyl]pyridin;
5-(trans-5-Dodecyl-1,3-dioxan-2-yl)-2-[4-heptylphenyl]pyridin;
5-(trans-5-Heptyl-1,3-dioxan-2-yl)-2-[4-octylphenyl]pyridin, Smp. (C-S) 55,5°C, S-S_{A} 145,7°C, Klp. (S_{A}-I) 173,7° C;
5-(trans-5-Octyl-1,3-dioxan-2-yl)-2-[4-octylphenyl]pyridin, Smp. (C-S) 58,8°C, S-S_{A} 145°C, Klp (S_{A}-I) 171°C;
5-(trans-5-Nonyl-1,3-dioxan-2-yl)-2-[4-octylphenyl]pyridin;
5-(trans-5-Decyl-1,3-dioxan-2-yl)-2-[4-octylphenyl]pyridin, Smp. (C-S) 64,0°C, S-S_{C} 144,5°C, S_{C}-S_{A} 156,5°C, Klp. (S_{A}-I) 167°C;
5-(trans-5-Dodecyl-1,3-dioxan-2-yl)-2-[4-octylphenyl]pyridin, Smp. (C-S) 71°C, S-S_{C} 143,5°C, Klp. (S_{C}-I) 165°C;
5-(trans-5-Nonyl-1,3-dioxan-2-yl)-2-[4-nonylphenyl]pyridin;
5-(trans-5-Decyl-1,3-dioxan-2-yl)-2-[4-nonylphenyl]pyridin;
5-)trans-5-Dodecyl-1,3-dioxan-2-yl)-2-[4-nonylphenyl]pyridin;
5-(trans-5-Decyl-1,3-dioxan-2-yl)-2-[4-decylphenyl]pyridin;
5-(trans-5-Dodecyl-1,3-dioxan-2-yl)-2-[4-decylphenyl]pyridin;
5-(trans-5-[10-Undecenyl]-1,3-dioxan-2-yl)-2-[4-heptylphenyl]pyridin;
5-(trans-5-[10-Undecenyl]-1,3-dioxan-2-yl)-2-[4-octylphenyl]pyridin;
5-(trans-5-Decyl-1,3-dioxan-2-yl)-2-[4-(hexyloxymethyl)phenyl]pyridin;
5-(trans-5-Octyl-1,3-dioxan-2-yl)-2-[3-fluor-4-octylphenyl]pyridin;
5-(trans-5-Decyl-1,3-dioxan-2-yl)-2-[3-fluor-4-octylphenyl]pyridin;
5-(trans-5-Dodecyl-1,3-dioxan-2-yl)-2-[3-fluor-4-octylphenyl]pyridin;
5-(trans-5-Octyl-1,3-dioxan-2-yl)-2-[3-fluor-4-nonylphenyl]pyridin;
5-(trans-5-Decyl-1,3-dioxan-2-yl)-2-[3-fluor-4-nonylphenyl]pyridin, Smp. (C-S) 54,9°C, S-S_{C} 99,7°C, S_{C}-S_{A} 136,5°C, Klp. (S_{A}-I) 153,5°C;
5-(trans-5-Dodecyl-1,3-dioxan-2-yl)-2-[3-fluor-4-nonylphenyl]pyridin, Smp. (C-S) 65,1°C, S-S_{C} 101,5°C, S_{C}-S_{A} 142,5°C, Klp. (S_{A}-I) 150°C;
5-(trans-5-Octyl-1,3-dioxan-2-yl)-2-[3-fluor-4-decylphenyl]pyridin;
5-(trans-5-Decyl-1,3-dioxan-2-yl)-2-[3-fluor-4-decylphenyl]pyridin;
5-(trans-5-Dodecyl-1,3-dioxan-2-yl)-2-[3-fluor-4-decylphenyl]pyridin;
5-[2-(trans-5-Octyl-1,3-dioxan-2-yl)äthyl]-2-[4-(heptyloxy)phenyl]pyridin;
5-[2-(trans-5-Decyl-1,3-dioxan-2-yl)äthyl]-2-[4-(heptyloxy)phenyl]pyridin;
5-[2-(trans-5-Octyl-1,3-dioxan-2-yl)äthyl]-2-[4-(octyloxy)phenyl]pyridin;
5-[2-(trans-5-Nonyl-1,3-dioxan-2-yl)äthyl]-2-[4-(octyloxy)phenyl]pyridin;
5-[2-(trans-5-Decyl-1,3-dioxan-2-yl)äthyl]-2-[4-(octyloxy)phenyl]pyridin;
5-[2-(trans-5-Octyl-1,3-dioxan-2-yl)äthyl]-2-[4-(nonyloxy)phenyl]pyridin;
5-[2-(trans-5-Decyl-1,3-dioxan-2-yl)äthyl]-2-[4-(nonyloxy)phenyl]pyridin;
5-[2-(trans-5-Octyl-1,3-dioxan-2-yl)äthyl]-2-[4-(decyloxy)phenyl]pyridin;
5-[2-(trans-5-Decyl-1,3-dioxan-2-yl)äthyl]-2-[4-(decyloxy)phenyl]pyridin;
5-[2-(trans-5-Decyl-1,3-dioxan-2-yl)äthyl]-2-[3-fluor-4-(heptyloxy)phenyl]pyridin;
5-[2-(trans-5-Dodecyl-1,3-dioxan-2-yl)äthyl]-2-[3-fluor-4-(heptyloxy)phenyl]pyridin;
5-[2-(trans-5-Octyl-1,3-dioxan-2-yl)äthyl]-2-[3-fluor-4-(octyloxy)phenyl]pyridin;
5-[2-(trans-5-Decyl-1,3-dioxan-2-yl)äthyl]-2-[3-fluor-4-(octyloxy)phenyl]pyridin;
5-[2-(trans-5-Dodecyl-1,3-dioxan-2-yl)äthyl]-2-[3-fluor-4-(octyloxy)phenyl]pyridin;
5-[2-(trans-5-Nonyl-1,3-dioxan-2-yl)äthyl]-2-[3-fluor-4-(decyloxy)phenyl]pyridin;
5-[2-(trans-5-Decyl-1,3-dioxan-2-yl)äthyl]-2-[3-fluor-4-(decyloxy)phenyl]pyridin, Smp. (C-S) 57,3°C, S-S 68°C, S-S_{C} 115,7°C, Klp. (S_{C}-I) 139,5°C;
5-[2-(trans-5-Dodecyl-1,3-dioxan-2-yl)äthyl]-2-[3-fluor-4-(decyloxy)phenyl]pyridin, Smp. (C-S) 65,7°C, S-S_{C} 114°C, Klp. (S_{C}-I) 138°C;
5-[2-(trans-5-Decyl-1,3-dioxan-2-yl)äthyl]-2-[4-heptylphenyl]pyridin;
5-[2-(trans-5-Dodecyl-1,3-dioxan-2-yl)äthyl]-2-[4-heptylphenyl]pyridin;
5-[2-(trans-5-Decyl-1,3-dioxan-2-yl)äthyl]-2-[4-octylphenyl]pyridin;
5-[2-(trans-5-Dodecyl-1,3-dioxan-2-yl)äthyl]-2-[4-octylphenyl]pyridin;
5-[2-(trans-5-Nonyl-1,3-dioxan-2-yl)äthyl]-2-[4-nonylphenyl]pyridin;
5-[2-(trans-5-Decyl-1,3-dioxan-2-yl)äthyl]-2-[4-nonylphenyl]pyridin;
5-[2-(trans-5-Dodecyl-1,3-dioxan-2-yl)äthyl]-2-[4-nonylphenyl]pyridin;
5-[2-(trans-5-Octyl-1,3-dioxan-2-yl)äthyl]-2-[4-decylphenyl]pyridin;
5-[2-(trans-5-Decyl-1,3-dioxan-2-yl)äthyl]-2-[4-decylphenyl]pyridin;
5-[2-(trans-5-Dodecyl-1,3-dioxan-2-yl)äthyl]-2-[4-decylphenyl]pyridin.

### Beispiel 2

a) Ein Gemisch aus 44,3 g 6-Chlor-3-pyridincarbinol, 300 ml Dioxan und 104,3 g 3,4-Dihydro-2H-pyran wurde mit 3 g p-Toluolsulfonsäure-Monohydrat versetzt und 2 Stunden bei 55°C gerührt. Das Reaktionsgemisch wurde mit 10 ml Diäthylamin versetzt und nach dem Erkalten in Diäthyläther aufgenommen. Mehrmaliges Waschen mit Wasser, Trocknen über Natriumsulfat und Einengen im Vakuum lieferte 87,3 g rohes 2-Chlor-5-[(tetrahydro-2-pyranyloxy)methyl]pyridin.
b) Eine aus 0,8 g Magnesium, 10,3 g (4-Bromphenyl)octyläther und 50 ml Tetrahydrofuran bereitete Grignard-Reagens-Lösung wurde bei 0 - 5°C innert 30 Minuten in ein Gemisch aus 7,5 g rohem 2-Chlor-5-[(tetrahydro-2-pyranyloxy)methyl]pyridin, 75 ml Tetrahydrofuran und 0,36 g 1,3-Bis(diphenylphosphino)propannickel-(II)-chlorid getropft. Das Reaktionsgemisch wurde 3 Stunden gerührt, über Nacht stehen gelassen, dann mit wässriger Natriumhydrogencarbonat-Lösung auf pH8 gestellt und mit Diäthyläther verdünnt. Die organische Phase wurde mit Wasser neutral gewaschen, über Natriumsulfat getrocknet und eingeengt. Chromatographische Reinigung des Rückstandes (14,6 g) an 210 g Kieselgel mit Hexan/Aethylacetat (Vol. 4:1) lieferte 8,8 g 2-[4-(Octyloxy)phenyl]-5-[(tetrahydro-2-pyranyloxy)methyl]pyridin.
c) Eine Lösung von 8,8 g 2-[4-(Octyloxy)phenyl]-5-[(tetrahydro-2-pyranyloxy)methyl]pyridin in 150 ml Tetrahydrofuran wurde mit 22 ml 2 N Salzsäure versetzt. Das Gemisch wurde 6 Stunden bei 60°C gerührt, dann mit Diäthyläther verdünnt, zweimal mit je 50 ml gesättigter wässriger Natriumhydrogencarbonat-Lösung und dreimal mit je 75 ml Wasser gewaschen, über Natriumsulfat getrocknet und eingeengt. Es resultierten 7,0 g 6-[4-(Octyloxy)phenyl]-3-pyridincarbinol.
d) Eine Lösung von 7,0 g 6-[4-(Octyloxy)phenyl]-3-pyridincarbinol in 150 ml 1,2-Dichloräthan wurde mit 9,6 g aktiviertem Mangan-(IV)-oxid versetzt. Die Suspension wurde 4,5 Stunden zum Sieden erhitzt und dann filtriert. Einengen des Filtrates ergab 6,2 g 6-[4-(Octyloxy)phenyl]-3-pyridincarboxyaldehyd; Smp. 90,5 - 91°C.

In analoger Weise können folgende Verbindungen hergestellt werden:
6-[4-(Hexyloxy)phenyl]-3-pyridincarboxaldehyd;
6-[4-(Heptyloxy)phenyl]-3-pyridincarboxaldehyd;
6-[4-(Nonyloxy)phenyl]-3-pyridincarboxaldehyd;
6-[4-(Decyloxy)phenyl]-3-pyridincarboxaldehyd;
6-[4-[(S)-4-Methylhexyloxy]phenyl]-3-pyridincarboxaldehyd;
6-[4-[(S)-5-Methylheptyloxy]phenyl]-3-pyridincarboxaldehyd;
6-[4-[(S)-6-Methyloctyloxy]phenyl]-3-pyridincarboxaldehyd;
6-[4-[(R)-2-Fluoroctyloxy]phenyl]-3-pyridincarboxaldehyd;
6-[4-[(S)-2-Fluoroctyloxy]phenyl]-3-pyridincarboxaldehyd;
6-[3-Fluor-4-(hexyloxy)phenyl]-3-pyridincarboxaldehyd;
6-[3-Fluor-4-(heptyloxy)phenyl]-3-pyridincarboxaldehyd;
6-[3-Fluor-4-(octyloxy)phenyl]-3-pyridincarboxaldehyd, Smp. 65,5 - 66°C;
6-[3-Fluor-4-(decyloxy)phenyl]-3-pyridincarboxaldehyd, Smp. 53 - 54,8°C;
6-[2-Fluor-4-(hexyloxy)phenyl]-3-pyridincarboxaldehyd;
6-[2-Fluor-4-(octyloxy)phenyl]-3-pyridincarboxaldehyd;
6-[2-Fluor-4-(decyloxy)phenyl]-3-pyridincarboxaldehyd;
6-[2,3-Difluor-4-(hexyloxy)phenyl]-3-pyridincarboxaldehyd;
6-[2,3-Difluor-4-(heptyloxy)phenyl]-3-pyridincarboxaldehyd;
6-[2,3-Difluor-4-(octyloxy)phenyl]-3-pyridincarboxaldehyd;
6-[2,3-Difluor-4-(nonyloxy)phenyl]-3-pyridincarboxaldehyd;
6-[2,3-Difluor-4-(decyloxy)phenyl]-3-pyridincarboxaldehyd;
6-[3-Chlor-4-(hexyloxy)phenyl]-3-pyridincarboxaldehyd;
6-[3-Chlor-4-(octyloxy)phenyl]-3-pyridincarboxaldehyd;
6-[3-Chlor-4-(decyloxy)phenyl]-3-pyridincarboxaldehyd;
6-[3-Methyl-4-(hexyloxy)phenyl)-3-pyridincarboxaldehyd;
6-[3-Methyl-4-(octyloxy)phenyl]-3-pyridincarboxaldehyd;
6-[3-Methyl-4-(decyloxy)phenyl]-3-pyridincarboxaldehyd;
6-[4-Heptylphenyl]-3-pyridincarboxaldehyd;
6-[4-Octylphenyl]-3-pyridincarboxaldehyd, Smp. 51,2-52,9°C;
6-[4-Nonylphenyl]-3-pyridincarboxaldehyd;
6-[4-Decylphenyl]-3-pyridincarboxaldehyd;
6-[4-(Hexyloxymethyl)phenyl]-3-pyridincarboxaldehyd;
6-[3-Fluor-4-octylphenyl]-3-pyridincarboxaldehyd;
6-[3-Fluor-4-nonylphenyl]-3-pyridincarboxaldehyd;
6-[3-Fluor-4-decylphenyl]-3-pyridincarboxaldehyd.

### Beispiel 3

a) Eine Suspension von 6,44 g (1,3-Dioxolan-2-yl)methyl-triphenylphosphoniumbromid in 70 ml Diäthyläther wird unter Stickstoffbegasung innert 5 Minuten mit 1,68 g Kalium-t-butylat versetzt. Die orange Suspension wird 30 Minuten gerührt, dann auf 0°C gekühlt und innert 10 Minuten mit einer Lösung von 3,11 g 6-[4-(Octyloxy)phenyl]-3-pyridincarboxaldehyd (hergestellt gemäss Beispiel 2) in 25 ml Diäthyläther versetzt. Das Reaktionsgemisch wird noch 3 Stunden bei Raumtemperatur gerührt und dann mit 0,6 g Natriumhydrogencarbonat und mit 20 ml Wasser versetzt. Die organische Phase wird mit Wasser gewaschen, über Natriumsulfat getrocknet und eingeengt. Der Rückstand wird in Hexan und 20 ml 80-prozentigem wässrigem Methanol aufgenommen. Die Phasen werden getrennt und die Hexan-Phase wird mit 10 ml 80-prozentigem Methanol gewaschen, über Natriumsulfat getrocknet und eingeengt. Chromatographische Reinigung des Rückstandes an 50 g Kieselgel mit Hexan/Aethylacetat (Vol. 9:1) ergibt 5-[2-(1,3-Dioxolan-2-yl)vinyl]-2-[4-(octyloxy)phenyl]pyridin.
b) Eine Lösung von 2,86 g 5-[2-(1,3-Dioxolan-2-yl)vinyl]-2-[4-(octyloxy)phenyl]pyridin in 50 ml Dioxan und 1 ml Triäthylamin wird mit 0,5 g 10-prozentiger Palladium/Kohle 2 Stunden hydriert. Filtration und Einengen des Filtrates ergibt rohes 5-[2-(1,3-Dioxolan-2-yl)äthyl]-2-[4-(octyloxy)phenyl]pyridin.
c) Eine Lösung von 2,68 g 5-[2-(1,3-Dioxolan-2-yl)äthyl]-2-[4-(octyloxy)phenyl]pyridin in 25 ml Toluol wird bei 0°C unter Stickstoffbegasung innert 10 Minuten mit 15 ml Ameisensäure versetzt. Das Zweiphasen-Gemisch wird über Nacht bei Raumtemperatur gerührt und dann vorsichtig auf 120 ml 2N wässrige Natriumcarbonat-Lösung gegossen. Das Gemisch wird dreimal mit je 50 ml Diäthyläther extrahiert. Die vereinigten Extrakte werden mit Wasser gewaschen, über Natriumsulfat getrocknet und filtriert. Einengen des Filtrates ergibt 3-[6-[4-(Octyloxy)phenyl]-3-pyridyl]propionaldehyd.

In analoger Weise können folgende Verbindungen hergestellt werden:
3-[6-[4-(Heptyloxy)phenyl]-3-pyridyl]propionaldehyd;
3-[6-[4-(Nonyloxy)phenyl]-3-pyridyl]propionaldehyd;
3-[6-[4-(Decyloxy)phenyl]-3-pyridyl]propionaldehyd;
3-[6-[3-Fluor-4-(heptyloxy)phenyl]-3-pyridyl]propionaldehyd;
3-[6-[3-Fluor-4-(octyloxy)phenyl]-3-pyridyl]propionaldehyd;
3-[6-[3-Fluor-4-(decyloxy)phenyl]-3-pyridyl]propionaldehyd;
3-[6-(4-Heptylphenyl)-3-pyridyl]propionaldehyd;
3-[6-(4-Octylphenyl)-3-pyridyl]propionaldehyd;
3-[6-(4-Nonylphenyl)-3-pyridyl]propionaldehyd;
3-[6-(4-Decylphenyl)-3-pyridyl]propionaldehyd.

### Beispiel 4

Die Verwendung der Verbindungen der Formel I in getilteten smektischen Mischungen wird durch die folgenden Mischungsbeispiele veranschaulicht. Die Ganghöhe (pitch) p wurde bei 25°C bestimmt. Die Schaltzeiten τ der ferroelektrischen Gemische wurden in einer Flüssigkristallzelle gemäss Appl. Phys. Lett. 36, 899 (1980) und 2 µm Plattenabstand und bei 20 V (peak-to-peak, Rechteckspannung, 100 Hz) und 25°C gemessen.

### Mischung A

6,63 Gew.-% 5-Heptyl-2-[p-(octyloxy)phenyl]pyrimidin,
4,50 Gew.-% 5-Heptyl-2-[p-(nonyloxy)phenyl]pyrimidin,
4,47 Gew.-% 5-Octyl-2-[p-(hexyloxy)phenyl]pyrimidin,
4,60 Gew.-% 5-Octyl-2-[p-(nonyloxy)phenyl]pyrimidin,
11,10 Gew.-% 5-Nonyl-2-[p-(hexyloxy)phenyl]pyrimidin,
22,22 Gew.-% 5-Nonyl-2-[p-(nonyloxy)phenyl]pyrimidin,
10,70 Gew.-% 4-(Decyloxy)-2,3-difluorbenzoesäure-p-[2-(trans-4-pentylcyclohexyl)äthyl]phenylester,
4,64 Gew.-% 4-(Heptyloxy)-2,3-difluorbenzoesäure-p-[trans-4-[(R)-2-fluorhexanoyloxy]cyclohexyl]phenylester,
4,29 Gew.-% 4-(Nonyloxy)-2,3-difluorbenzoesäure-p-[trans-4-[(R)-2-fluorhexanoyloxy]cyclohexyl]phenylester,
3,66 Gew.-% 5-(trans-5-Heptyl-1,3-dioxan-2-yl)-2-(4-octylphenyl)pyridin,
3,74 Gew.-% 5-(trans-5-Octyl-1,3-dioxan-2-yl)-2-[4-octylphenyl)pyridin,
1,88 Gew.-% 5-(trans-5-Decyl-1,3-dioxan-2-yl)-2-(4-octylphenyl)pyridin,
2,60 Gew.-% 5-(trans-5-Heptyl-1,3-dioxan-2-yl)-2-[4-(octyloxy)phenyl]pyridin,
3,00 Gew.-% 5-(trans-5-Octyl-1,3-dioxan-2-yl)-2-[4-(octyloxy)phenyl]pyridin,
3,04 Gew.-% 5-(trans-5-Decyl-1,3-dioxan-2-yl)-2-[4-(octyloxy)phenyl]pyridin,
4,38 Gew.-% 5-(trans-5-Heptyl-1,3-dioxan-2-yl)-2-[3-fluor-4-(octyloxy)phenyl]pyridin,
4,55 Gew.-% 5-(trans-5-Decyl-1,3-dioxan-2-yl)-2-[3-fluor-4-(octyloxy)phenyl]pyridin;
S_{C}*-S_{A} 77°C, S_{A}-N* 87°C, Klp. (N*-I) 98°C; Kristallisation (S_{C}*-C) <0°C; p = 16 µm, τ = 155 µs.

### Mischung B

6,15 Gew.-% 5-Heptyl-2-[p-(octyloxy)phenyl]pyrimidin,
4,18 Gew.-% 5-Heptyl-2-[p-(nonyloxy)phenyl]pyrimidin,
4,44 Gew.-% 5-Octyl-2-[p-(hexyloxy)phenyl]pyrimidin,
4,28 Gew.-% 5-Octyl-2-[p-(nonyloxy)phenyl]pyrimidin,
10,48 Gew.-% 5-Nonyl-2-[p-(hexyloxy)phenyl]pyrimidin,
20,93 Gew.-% 5-Nonyl-2-[p-(nonyloxy)phenyl]pyrimidin,
3,89 Gew.-% trans-5-Heptyl-2-[4′-(10-undecenyloxy)-4-biphenylyl]-1,3-dioxan,
3,83 Gew.-% trans-5-Octyl-2-[4′-(10-undecenyloxy)-4-biphenylyl]-1,3-dioxan,
1,89 Gew.-% trans-5-Decyl-2-[4′-(10-undecenyloxy)-4-biphenylyl]-1,3-dioxan,
10,00 Gew.-% 4-(Decyloxy)-2,3-difluorbenzoesäure-p-[2-(trans-4-propylcyclohexyl)äthyl]phenylester,
5,30 Gew.-% 4-(Heptyloxy)-2,3-difluorbenzoesäure-p-[trans-4-[(R)-2-fluorhexanoyloxy]cyclohexyl]phenylester,
5,00 Gew.-% 4-(Nonyloxy)-2,3-difluorbenzoesäure-p-[trans-4-[(R)-2-fluorhexanoyloxy]cyclohexyl]phenylester,
2,99 Gew.-% 5-(trans-5-Heptyl-1,3-dioxan-2-yl)-2-[4-(octyloxy)phenyl]pyridin,
3,51 Gew.-% 5-(trans-5-Octyl-1,3-dioxan-2-yl)-2-[4-(octyloxy)phenyl]pyridin,
3,51 Gew.-% 5-(trans-5-Decyl-1,3-dioxan-2-yl)-2-[4-(octyloxy)phenyl]pyridin,
4,71 Gew.-% 5-(trans-5-Heptyl-1,3-dioxan-2-yl)-2-[3-fluor-4-(octyloxy)phenyl]pyridin,
4,91 Gew.-% 5-(trans-5-Decyl-1,3-dioxan-2-yl)-2-[3-fluor-4-(octyloxy)phenyl]pyridin;
S_{C}*-S_{A} 80°C, S_{A}-N* 86,5°C, Klp. (N*-I) 102°C;
Kristallisation (S_{C}*-C) -3°C; p = 14 µm, τ = 160 µs.

### Mischung C

4,94 Gew.-% 5-Heptyl-2-[p-(octyloxy)phenyl]pyrimidin,
8,33 Gew.-% 5-Octyl-2-[p-(hexyloxy)phenyl]pyrimidin,
7,54 Gew.-% 5-Octyl-2-[p-(nonyloxy)phenyl]pyrimidin,
10,71 Gew.-% 5-Nonyl-2-[p-(hexyloxy)phenyl]pyrimidin,
22,34 Gew.-% 5-Nonyl-2-[p-(nonyloxy)phenyl]pyrimidin,
6,01 Gew.-% 5-[(S)-5-Methylheptyl]-2-[p-(10-undecenyloxy)phenyl]pyrimidin,
9,53 Gew.-% trans-5-Octyl-2-[3′-fluor-4′-(octyloxy)-4-biphenylyl]-1,3-dioxan,
5,02 Gew.-% 4-(Heptyloxy)-2,3-difluorbenzoesäure-p-[trans-4-[(R)-2-fluorhexanoyloxy]cyclohexyl]phenylester,
4,82 Gew.-% 4-(Nonyloxy)-2,3-difluorbenzoesäure-p-[trans-4-[(R)-2-fluorhexanoyloxy]cyclohexyl]phenylester,
1,82 Gew.-% 5-(trans-5-Heptyl-1,3-dioxan-2-yl)-2-(4-octylphenyl)pyridin,
1,86 Gew.-% 5-(trans-5-Octyl-1,3-dioxan-2-yl)-2-(4-octylphenyl)pyridin,
0,94 Gew.-% 5-(trans-5-Decyl-1,3-dioxan-2-yl)-2-(4-octylphenyl)pyridin,
1,74 Gew.-% 5-(trans-5-Heptyl-1,3-dioxan-2-yl)-2-[4-(octyloxy)phenyl]pyridin,
2,04 Gew.-% 5-(trans-5-Octyl-1,3-dioxan-2-yl)-2-[4-(octyloxy)phenyl]pyridin,
2,03 Gew.-% 5-(trans-5-Decyl-1,3-dioxan-2-yl)-2-[4-(octyloxy)phenyl]pyridin;
5,06 Gew.-% 5-(trans-5-Heptyl-1,3-dioxan-2-yl)-2-[3-fluor-4-(octyloxy)phenyl]pyridin;
4,82 Gew.-% 5-(trans-5-Decyl-1,3-dioxan-2-yl)-2-[3-fluor-4-(octyloxy)phenyl]pyridin;
S_{C}*-S_{A} 76°C, Klp. (S_{A}-I) 100°C; Kristallisation (S_{C}*-C) <O°C.

### Mischung D

6,63 Gew.-% 5-Heptyl-2-[p-(octyloxy)phenyl]pyrimidin,
4,50 Gew.-% 5-Heptyl-2-[p-(nonyloxy)phenyl]pyrimidin,
4,47 Gew.-% 5-Octyl-2-[p-[hexyloxy)phenyl]pyrimidin,
4,60 Gew.-% 5-Octyl-2-[p-(nonyloxy)phenyl]pyrimidin,
11,10 Gew.-% 5-Nonyl-2-[p-(hexyloxy)phenyl]pyrimidin,
22,22 Gew.-% 5-Nonyl-2-[p-(nonyloxy)phenyl]pyrimidin,
2,48 Gew.-% trans-5-Nonyl-2-[4′-(octyloxy)-4-biphenylyl]-1,3-dioxan,
2,58 Gew.-% trans-5-Heptyl-2-[4′-(8-nonenyloxy)-4-biphenylyl]-1,3-dioxan,
2,47 Gew.-% 4-(Dodecyloxy)benzoesäure-p-[2-(trans-4-pentylcyclohexyl)äthyl]phenylester,
4,88 Gew.-% 4-(Dodecyloxy)-2-fluorbenzoesäure-p-[2-(trans-4-pentylcyclohexyl)äthyl]phenylester,
5,00 Gew.-% 4-(Decyloxy)-2,3-difluorbenzoesäure-p-[2-(trans-4-propylcyclohexyl)äthyl]phenylester,
4,64 Gew.-% 4-(Heptyloxy)-2,3-difluorbenzoesäure-p-[trans-4-[(R)-2-fluorhexanoyloxy]cyclohexyl]phenylester,
4,29 Gew.-% 4-(Nonyloxy)-2,3-difluorbenzoesäure-p-[trans-4-[(R)-2-fluorhexanoyloxy]cyclohexyl]phenylester,
11,08 Gew.-% 5-(trans-5-Heptyl-1,3-dioxan-2-yl)-2-[3-fluor-4-(octyloxy)phenyl]pyridin,
9,06 Gew.-% 5-(trans-5-Decyl-1,3-dioxan-2-yl)-2-[3-fluor-4-(octyloxy)phenyl]pyridin; S_{C}*-S_{A} 73,5°C, S_{A}-N* 87°C, Klp. (N*-I) 95,5°C; Kristallisation (S_{C}*-C) <0°C; τ = 240 µs.

### Mischung E

29,39 Gew.-% 5-Nonyl-2-[p-(hexyloxy)phenyl]pyrimidin,
29,96 Gew.-% 5-Nonyl-2-[p-(nonyloxy)phenyl]pyrimidin,
8,54 Gew.-% 4-(Nonyloxy)-2,3-difluorbenzoesäure-p-[trans-4-[(R)-2-fluorhexanoyloxy]cyclohexyl]phenylester,
4,63 Gew.-% 5-Nonyl-2-(4-[(trans-4-[(R)-2-fluorhexanoyloxy]cyclohexyl)methoxy]phenyl)pyrimidin,
10,59 Gew.-% trans-5-Heptyl-2-[4′-(6-heptenyloxy)-4-biphenylyl]-1,3-dioxan
7,63 Gew.-% 5-(trans-5-Heptyl-1,3-dioxan-2-yl)-2-[4-(octyloxy)phenyl]pyridin,
9,26 Gew.-% 5-(trans-5-Octyl-1,3-dioxan-2-yl)-2-[4-(octyloxy)phenyl]pyridin;
S_{C}*-S_{A} 79°C, S_{A}-N* 91°C, Klp. (N*-I) 98°C; τ = 175 µs.

### Mischung F (Vergleichsmischung)

29,39 Gew.-% 5-Nonyl-2-[p-(hexyloxy)phenyl]pyrimidin,
29,96 Gew.-% 5-Nonyl-2-[p-(nonyloxy)phenyl]pyrimidin, 8,54 Gew.-% 4-(Nonyloxy)-2,3-difluorbenzoesäure-p-[trans-4-[(R)-2-fluorhexanoyloxy]cyclohexyl]phenylester,
4,33 Gew.-% 5-Nonyl-2-(4-[(trans-4-[(R)-2-fluorhexanoyloxy]cyclohexyl)methoxy]phenyl)pyrimidin,
7,46 Gew.-% 4-(Decyloxy)benzoesäure-p-[2-(trans-4-pentylcyclohexyl)äthyl]phenylester,
7,30 Gew.-% 4-( Dodecyloxy)benzoesäure-p-[2-(trans-4-pentylcyclohexyl)äthyl]phenylester,
13,02 Gew.-% 4-(Dodecyloxy-2-fluorbenzoesäure-p-[2-(trans-4-pentylcyclohexyl)äthyl]phenylester;
S_{C*}-S_{A} 59°C, S_{A}-N* 68°C, Klp. (N*-I) 87°C; τ = 300 µs.

Die Verwendung der vergleichsweise niederviskosen, dreikernigen S_{C}-Komponenten von Typ 4-(Alkoxy)benzoesäure-p-[2-(trans-4-alkylcyclohexyl)äthyl]phenylester anstelle von erfindungsgemässen Verbindungen ergibt im Vergleich zu Mischung E einen tieferen Phasenübergang S_{C}*-S_{A} und eine längere Schaltzeit.

## Patentansprüche

1. Verbindungen der allgemeinen Formel 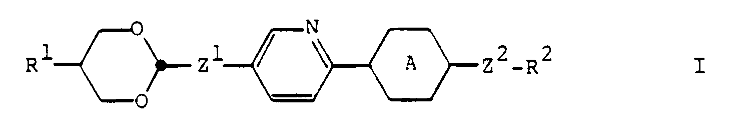 worin R¹ Alkyl oder Alkenyl bedeutet; Z¹ eine einfache Kovalenzbindung oder -CH₂CH₂- bezeichnet; Ring A unsubstitutiertes oder mit Halogen und/oder Methyl substituiertes 1,4-Phenylen darstellt; Z² eine einfache Kovalenzbindung, Sauerstoff oder -CH₂O- bezeichnet; und R² eine unsubstituierte oder mit Halogen substituierte Alkyl- oder Alkenylgruppe bedeutet.

2. Verbindungen nach Anspruch 1 gekennzeichnet durch die allgemeine Formel 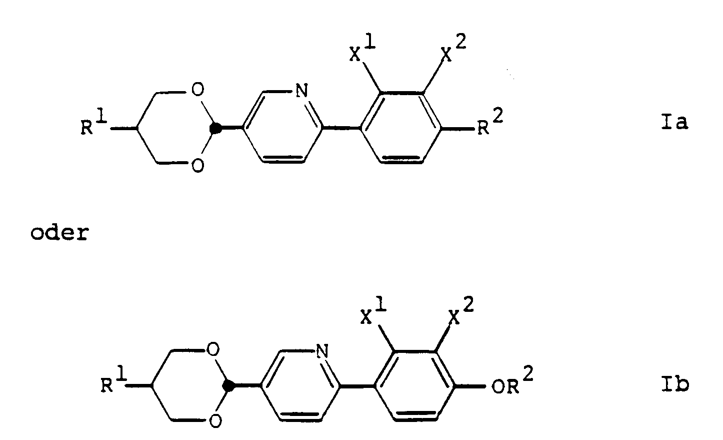 worin R¹ und R² die in Anspruch 1 gegebenen Bedeutungen haben, und X¹ und X² unabhängig voneinander Wasserstoff, dalogen oder Methyl bezeichnen.

3. Verbindungen nach Anspruch 2, dadurch gekennzeichnet, dass X¹ und X² unabhängig voneinander Wasserstoff oder Fluor bedeuten.

4. Verbindungen nach einem der Ansprüche 1 bis 3, dadurch gekennzeichnet, dass R¹ 3 - 14 Kohlenstoffatome aufweist.

5. Verbindungen nach einem der Ansprüche 1 bis 4, dadurch gekennzeichnet, dass R² 3 - 12 Kohlenstoffatome aufweist.

6. Verbindungen nach einem der Ansprüche 1 bis 5, dadurch gekennzeichnet, dass R¹ 1E-Alkenyl, 3E-Alkenyl, Alkenyl mit endständiger Doppelbindung oder Alkyl bedeutet.

7. Verbindungen nach einem der Ansprüche 1 bis 6, dadurch gekennzeichnet, dass R² einen unsubstituierten oder mit Halogen substituierten Rest aus der Gruppe Alkyl, 3E-Alkenyl und Alkenyl mit endständiger Doppelbindung bedeutet, wenn Z² eine einfache Kovalenzbindung bezeichnet, bzw. R² einen unsubstituierten oder mit Halogen substituierten Rest aus der Gruppe Alkyl, 2E-Alkenyl und Alkenyl mit endständiger Doppelbindung bedeutet, wenn Z² Sauerstoff bezeichnet, bzw. R² einen unsubstituierten oder mit Halogen substituierten Rest aus der Gruppe Alkyl und Alkenyl mit endständiger Doppelbindung bedeutet, wenn Z² -CH₂O- bezeichnet.

8. Flüssigkristallines Gemisch mit mindestens 2 Komponenten, dadurch gekennzeichnet, dass mindestens eine Komponente eine Verbindung der in Anspruch 1 definierten Formel I ist.

9. Verfahren zur Herstellung der Verbindungen der in Anspruch 1 definierten Formel I, dadurch gekennzeichnet, dass man einen Aldehyd der allgemeinen Formel 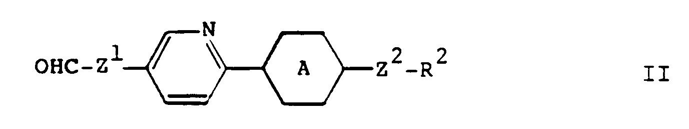 oder ein Acetal hiervon mit einer Verbindung der allgemeinen Formel 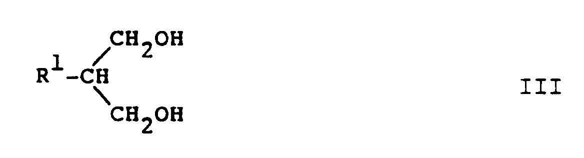 worin R¹, R², Z¹, Z² und Ring A die in Anspruch 1 gegebenen Bedeutungen haben, umsetzt.

10. Verwendung der Verbindungen der in Anspruch 1 definierten Formel I für elektro-optische Zwecke.

## Claims

1. Compounds of the general formula 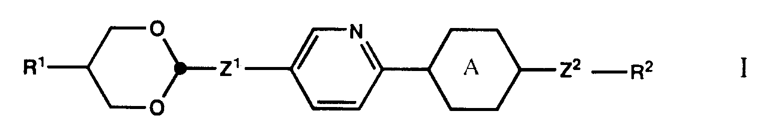 wherein R¹ signifies alkyl or alkenyl; Z¹ denotes a single covalent bond or -CH₂CH₂-; ring A represents unsubstituted or halogen- and/or methyl-substituted 1,4-phenylene; Z² denotes a single covalent bond, oxygen or -CH₂O-; and R² signifies an unsubstituted or halogen-substituted alkyl or alkenyl group.

2. Compounds according to claim 1, characterized by the general formula 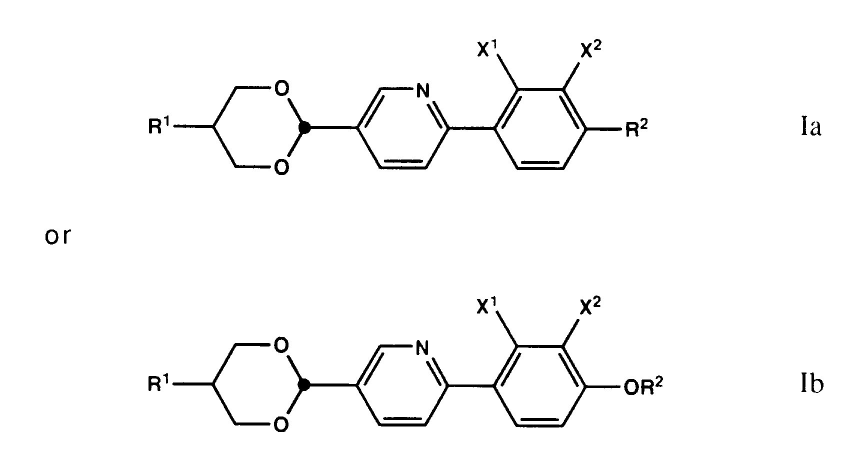 wherein R¹ and R² have the significances given in claim 1 and X¹ and X² each independently denote hydrogen, halogen or methyl.

3. Compounds according to claim 2, characterized in that X¹ and X² each independently signify hydrogen or fluorine.

4. Compounds according to any one of claims 1 to 3, characterized in that R¹ has 3-14 carbon atoms.

5. Compounds according to any one of claims 1 to 4, characterized in that R² has 3-12 carbon atoms.

6. Compounds according to any one of claims 1 to 5, characterized in that R¹ signifies 1E-alkenyl, 3E-alkenyl, alkenyl with a terminal double bond or alkyl.

7. Compounds according to any one of claims 1 to 6, characterized in that R² signifies an unsubstituted or halogen-substituted residue from the group alkyl, 3E-alkenyl and alkenyl with a terminal double bond when Z² denotes a single covalent bond or R² signifies an unsubstituted or halogen-substituted residue from the group alkyl, 2E-alkenyl and alkenyl with a terminal double bond when Z² denotes oxygen or R² signifies an unsubstituted or halogen-substituted residue from the group alkyl and alkenyl with a terminal double bond when Z² denotes -CH₂O-.

8. A liquid crystalline mixture with at least 2 components, characterized in that at one component is a compound of formula I defined in claim 1.

9. A process for the manufacture of the compounds of formula I defined in claim 1, characterized by reacting an aldehyde of the general formula 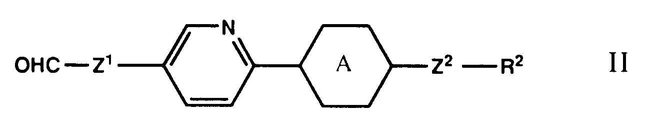 or an acetal thereof with a compound of the general formula 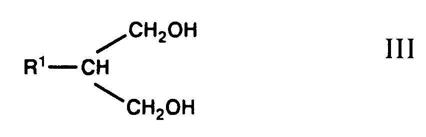 wherein R¹, R², Z¹, Z² and ring A have the significances given in claim 1.

10. The use of the compounds of formula I defined in claim 1 for electro-optical purposes.

## Revendications

1. Composés de formule générale 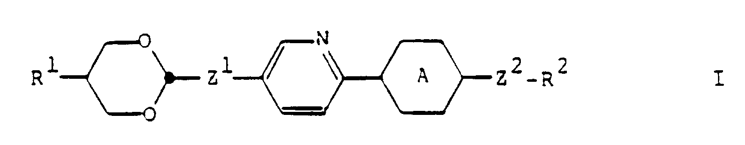 dans laquelle R¹ représente un groupe alkyle ou alcényle; Z¹ représente une simple liaison covalente ou -CH₂CH₂-; le cycle A représente un cycle 1,4-phénylène non substitué ou substitué par des substituants halogène et/ou méthyle; Z² représente une simple liaison covalente, un atome d'oxygène ou -CH₂O-; et R² représente un groupe alkyle ou alcényle non subtitué ou substitué par un atome d'halogène.

2. Composés selon la revendication 1, caractérisés par la formule générale 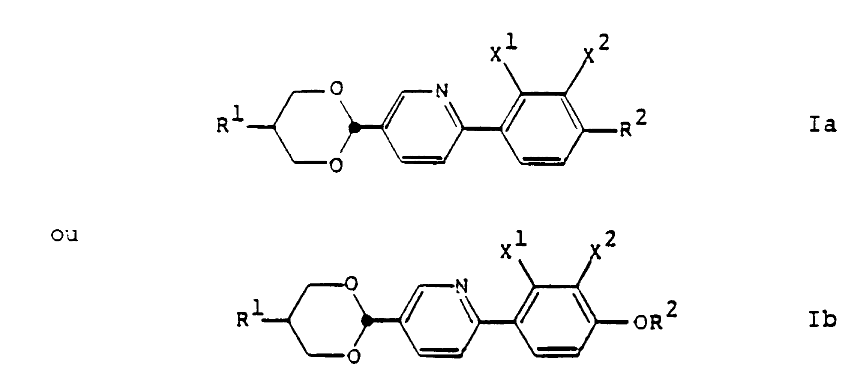 formules dans lesquelles R¹ et R² ont les significations données dans la revendication 1, et X¹ et X² représentent, indépendamment l'un de l'autre, un atome d'hydrogène ou d'halogène ou le groupe méthyle.

3. Composés selon la revendication 2, caractérisés en ce que X¹ et X² représentent, indépendamment l'un de l'autre, un atome d'hydrogène ou de fluor.

4. Composés selon l'une des revendications 1 à 3, caractérisés en ce que R¹ comporte 3-14 atomes de carbone.

5. Composés selon l'une des revendications 1 à 4, caractérisés en ce que R² comporte 3-12 atomes de carbone.

6. Composés selon l'une des revendications 1 à 5, caractérisés en ce que R¹ représente un groupe 1E-alcényle, 3E-alcényle, alcényle à double liaison en fin de chaîne ou alkyle.

7. Composés selon l'une des revendications 1 à 6, caractérisés en ce que R² représente un radical, non substitué ou substitué par un atome d'halogène, choisi parmi un radical alkyle, un radical 3E-alcényle et un radical alcényle à double liaison en fin de chaîne, lorsque Z² représente une simple liaison covalente, ou R² représente un radical, non substitué ou substitué par un atome d'halogène, choisi parmi un radical alkyle, un radical 2E-alcényle et un radical alcényle à double liaison en fin de chaîne, lorsque Z² représente un atome d'oxygène, ou R² représente un radical, non substitué ou substitué par un atome d'halogène, choisi parmi un radical alkyle et un radical alcényle à double liaison en fin de chaîne, lorsque Z² représente -CH₂O-.

8. Composition de cristaux liquides à au moins deux composants, caractérisée en ce qu'au moins un composant est un composé de formule I définie dans la revendication 1.

9. Procédé pour la préparation des composés de formule I définie dans la revendication 1, caractérisé en ce que l'on fait réagir un aldéhyde de formule générale 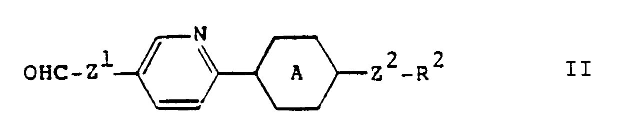 ou un acétal de celui-ci, avec un composé de formule générale 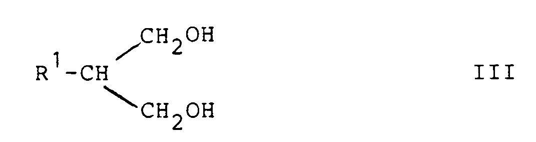 dans laquelle R¹, R², Z¹, Z² et le cycle A ont les significations données dans la revendication 1.

10. Utilisation des composés de formule I définie dans la revendication 1, à des fins électro-optiques.
